# EUROPEAN PATENT APPLICATION

(11) **EP 3 901 245 A1**
(43) Date of publication of application: **27.10.2021**
(21) Application number: 21163365.6
(22) Date of filing: 18.03.2021
(51) Int. Cl.: C12M 3/00, C12M 1/00, C12M 1/42

(54) **CELL CONTAINER AND METHOD FOR PRODUCING SAME**

(30) Priority: 30.03.2020 JP 2020061251; 26.01.2021 JP 2021010564
(71) Applicant: Ricoh Company, Ltd., Tokyo 143-8555 (JP)
(72) Inventor: NAKAYAMA, Tomoaki, Tokyo, tokyo 143-8555 (JP); ARATANI, Tomoyuki, Tokyo, Tokyo 143-8555 (JP); KOSHIZUKA, Shinnosuke, Tokyo, Tokyo 143-8555 (JP); MIYAOKA, Atsushi, Tokyo, Tokyo 143-8555 (JP); SHIONOIRI, Momoko, Tokyo, Tokyo 143-8555 (JP); YAMOTO, Rie, Tokyo, Tokyo 143-8555 (JP); KITAZAWA, Tomofumi, Tokyo, Tokyo 143-8555 (JP)
(74) Representative: SSM Sandmair

(57) **Abstract**

Provided is a cell-containing container including nerve cells and a medium, in which the nerve cells adhere to a culture surface of the cell-containing container, an adhesion area between the nerve cells and the culture surface is 0.5 mm² or more per 80,000 nerve cells, and a concentration of glucose in the medium is 1 g/L or higher.

## Description

### BACKGROUND OF THE INVENTION

### Field of the Invention

The present invention relates to a cell-containing container and a method for producing the same. Priorities are claimed on Japanese Patent Application No. 2020-061251, filed on March 30, 2020, and Japanese Patent Application No. 2021-010564, filed on January 26, 2021, the content of which are incorporated herein by reference.

### Description of Related Art

An action potential of nerve cells may be used to evaluate efficacy or toxicity with respect to the nerve cells. As one of methods for detecting and evaluating the action potential of the nerve cells, an evaluation method using a Microelectrode Array (MEA) is known. The MEA is an array of tiny electrodes placed on a substrate on which cells are cultured, and can detect an electrical activity of cells.

However, it is known that when the nerve cells derived from human iPS cells are cultured on the MEA and the action potential is detected, it is necessary to culture the nerve cells at higher density and for a longer period of time than in a case of using nerve cells derived from animals other than human.

### SUMMARY OF THE INVENTION

The inventors have found that when iPS-derived nerve cells are cultured at high density for a long period of time, cells may aggregate and detach from a culture surface of a culture container.

For example, Patent Document 1 (Japanese Unexamined Patent Application, First Publication No. 2018-117567) describes a cell culture apparatus having an object of appropriately maintaining and stabilizing a culture environment for living cells. The cell culture apparatus includes a culture tank for culturing living cells, an online/in-line monitoring device, a cell state determination device having a sterile sampling device, an analyzer, a data collection device, a data analyzer, and a cluster analysis function, and an operation control compensator having a reference function of cell reaction model for each cluster or the like. However, Patent Document 1 does not describe that nerve cells aggregate in a case where the nerve cells are cultured at high density for a long period of time, and also not describes a specific parameter necessary for suppressing such aggregation of nerve cells.

An object of the present invention is to provide a technique for suppressing aggregation of nerve cells.

A cell-containing container according to the present invention includes: nerve cells; and a medium, in which the nerve cells adhere to a culture surface of the cell-containing container, an adhesion area between the nerve cells and the culture surface is 0.949 to 28.2 mm² per 80,000 nerve cells, and a concentration of glucose in the medium is 1 g/L or higher.

A method for producing a cell-containing container according to the present invention includes a step of incubating a container including nerve cells and a medium, under a culture condition, while replacing the medium at a predetermined timing, in which a concentration of glucose in the medium is maintained at 1 g/L or higher for a predetermined period.

According to the present invention, it is possible to provide a technique for suppressing aggregation of nerve cells.

### BRIEF DESCRIPTION OF THE DRAWINGS

FIG. 1 is a graph showing results of measuring changes over time in glucose concentration in a nerve cell medium in Experimental Example 1.
FIGS. 2A to 2C show representative micrographs obtained by imaging nerve cells at 53rd day (DIV53) from seeding of nerve cells in Experimental Example 2.
FIG. 3A shows a representative micrograph of nerve cells evaluated as Aggregation Level 1 in Experimental Example 3. FIG. 3B shows a representative micrograph of nerve cells evaluated as Aggregation Level 2 in Experimental Example 3. FIG. 3C shows a representative micrograph of nerve cells evaluated as Aggregation Level 3 in Experimental Example 3. FIG. 3D shows a representative micrograph of nerve cells evaluated as Aggregation Level 4 in Experimental Example 3.
FIG. 4 is a graph showing changes over time in an aggregation level of nerve cells in Experimental Example 4.
FIG. 5 is a graph showing results obtained by examining a relationship between glucose concentration in a medium and an aggregation level of nerve cells in Experimental Example 5.

### DETAILED DESCRIPTION OF THE INVENTION

### [Cell-containing container]

The present invention provides a cell-containing container according to one embodiment including: nerve cells; and a medium, in which the nerve cells adhere to a culture surface of the cell-containing container, an adhesion area between the nerve cells and the culture surface is 0.5 mm² or more per 80,000 nerve cells, and a concentration of glucose in the medium is 1 g/L or higher.

As described below in Examples, the inventors have found that there is a relationship between aggregation of nerve cells and glucose concentration in a medium. In addition, it was clarified that in a case where nerve cells are cultured according to a normal protocol, the glucose concentration in the medium may be less than 1 g/L. Also, it was clarified that the aggregation of nerve cells can be suppressed by maintaining the glucose concentration in the medium at 1 g/L or more.

When the aggregation of nerve cells is suppressed, an action potential of nerve cells can be satisfactorily detected and evaluated using MEA or the like. Also, when the aggregation of nerve cells is suppressed, a state of nerve cells can be satisfactorily evaluated by other analysis means such as immunostaining.

The cell-containing container of the present embodiment is a culture of nerve cells in a culture container. The culture container may be a container generally used for cell culture, and a dish and a well plate are exemplary examples thereof. A diameter of the dish, the number of wells in the well plate, and the like can be appropriately selected according to an application.

In the cell-containing container of the present embodiment, an electrode array may be placed on a culture surface of the container. That is, the cell-containing container of the present embodiment may be a MEA plate. The number of electrodes in MEA and the like can be appropriately selected according to an application.

An organic material and an inorganic material described below are exemplary examples of a material of the culture surface of the culture container. One kind of these may be used alone and two or more kinds thereof may be used in combination.

The organic material is not particularly limited and can be appropriately selected according to the purpose. Polyethylene terephthalate (PET), polystyrene (PS), polycarbonate (PC), triacetyl cellulose (TAC), polyimide (PI), Nylon (Ny), low density polyethylene (LDPE), medium density polyethylene (MDPE), vinyl chloride, vinylidene chloride, polyphenylene sulfide, polyether sulfone, polyethylene naphthalate, polypropylene, an acrylic material such as urethane acrylate, cellulose, a silicone-based material such as polydimethylsiloxane (PDMS), polyvinyl alcohol (PVA), metal alginate salts such as calcium alginate, polyacrylamide, methylcellulose, and a gel-like material such as agarose are exemplary examples.

The inorganic material is not particularly limited and may be appropriately selected according to the purpose, and glass and ceramics are exemplary examples thereof.

The culture surface of the culture container may be coated with a coating agent. The coating agent usually used for cell culture can be appropriately used, and collagen, Matrigel (registered trademark, Corning), Geltrex (Thermo Fisher Scientific), PLO (Sigma-Aldrich), PDLO (Sigma-Aldrich), fibronectin, fibrinogen, gelatin, polyethyleneimine (PEI), laminin, and the like are exemplary examples thereof.

The nerve cell contained in the cell-containing container of the present embodiment may be a cell collected from a living body or a cell that has been established and cultured. In addition, from the viewpoint that it is easy to obtain a desired cell population containing a large amount of nerve cells, the cells may be differentiated from stem cells. That is, the nerve cells may be derived from stem cells.

Embryonic stem cells (ES cells), induced pluripotent stem cells, mesenchymal stem cells, cord blood-derived stem cells, nerve stem cells, and the like are exemplary examples of stem cells. Nuclear-transplanted embryonic stem cells (ntES cells), induced pluripotent stem cells (iPS cells), and the like are exemplary examples of induced pluripotent stem cells. Bone marrow mesenchymal stem cells, adipose tissue-derived mesenchymal stem cells, and the like are exemplary examples of mesenchymal stem cells. Among them, the stem cells are preferably iPS cells.

The iPS cells may be derived from a healthy person or a patient having various nervous system diseases. In addition, the cells that have been subjected to various gene edits may be used. For example, cells that have been engineered to have a gene that is a cause or risk factor of various nervous system diseases by gene edits may be used.

In a case where the iPS cells are derived from a patient with various nervous system diseases, the iPS cells can be used to construct a disease model of the nervous systems. A nervous system disease is not particularly limited, and neurodegenerative diseases, autism, epilepsy, attention-deficit hyperactivity disorder (ADHD), schizophrenia, bipolar disorder, and the like are exemplary examples thereof. Alzheimer's disease, Parkinson's disease, amyotrophic lateral sclerosis, and the like are exemplary examples of the neurodegenerative diseases.

Animal species from which the nerve cells are derived are not particularly limited, and humans, monkeys, dogs, cows, horses, sheep, pigs, rabbits, mice, rats, guinea pigs, and hamsters are exemplary examples thereof. Among these, humans are preferable.

Also, one kind of the nerve cells may be used alone or a mixture of two or more kinds of nerve cells may be used. The nerve cells can be roughly classified into, for example, peripheral nerves and central nerves. Sensory nerve cells, motor nerve cells, and autonomic nerve cells are exemplary examples of peripheral nerves. Intervening nerve cells and projection neurons are exemplary examples of the central nerves. Cortical neurons, hippocampal neurons, amygdala neurons, and the like are exemplary examples of the projection neurons. In addition, the central nerve cells can be roughly classified into excitatory neurons and inhibitory neurons. Glutamic acid-operated neurons mainly responsible for excitatory transmission in the central nervous system, GABA (γ-aminobutyric acid)-operated neurons mainly responsible for inhibitory transmission, and the like are exemplary examples thereof.

Cholinergic neurons, dopaminergic neurons, noradrenalinergic neurons, serotonergic neurons, histaminergic neurons, and the like are exemplary examples of other neurons that release neuromodulators.

The cell-containing container of the present embodiment may contain astrocytes, microglia, and the like together with nerve cells. An adhesion area between the nerve cells and the culture surface of the culture container is 0.5 mm² or more, preferably 0.949 mm² or more, more preferably 3 mm² or more, and still more preferably 3.14 mm² or more, per 80,000 nerve cells. Also, an upper limit of the adhesion area between the nerve cells and the culture surface of the culture container is preferably about 28.2 mm².

In addition, it is preferable that the nerve cells are mature depending on the purpose. For example, it is preferable that the expression of one the marker gene of Tubulin beta3, MAP2, NeuN, 160 kDa Neurofilament, 200 kDa Neurofilament, NSE, PSD93, and PSD95 is positive.

As the medium contained in the cell-containing container of the present embodiment, a medium suitable for the cells to be used can be appropriately selected and used, as long as the concentration of glucose in the medium is 1 g/L or higher.

A medium in which a necessary component is added to a basal medium is a specific exemplary examples of the medium. Dulbecco's Modified Eagle's Medium (DMEM), Ham F12 medium (Ham's Nutrient Mixture F12), D-MEM/F12 medium, McCoy's 5A medium, Eagle's MDM medium (Eagle's Minimum Essential Medium, EMEM), αMEM medium (alpha Modified Eagle's Medium Essential Medium, αMEM), MEM medium (Minimum Essential Medium), RPMI1640 (Roswell Park Memorial Institute-1640) medium, Iscove's Modified Dulbecco's Medium (IMDM), MCDB131 medium, William Medium E, IPL41 medium, Fischer's medium, M199 medium, High Performance Medium 199, StemPro34 (manufactured by Thermo Fisher Scientific), X-VIVO 10 (manufactured by Chembrex), X-VIVO 15 (manufactured by Chembrex), HPGM (manufactured by Chembrex), StemSpan H3000 (manufactured by Stem Cell Technologies), StemSpan SFEM (manufactured by Stem Cell Technologies), Stemline II (manufactured by Sigma-Aldrich), QBSF-60 (manufactured by Quality Biological), StemPro hESC SFM (manufactured by Thermo Fisher Scientific), Essential8 (registered trademark) medium (manufactured by Thermo Fisher Scientific), mTeSR1 or mTeSR2 medium (manufactured by Stem Cell Technologies), Repro FF or Repro FF2 (manufactured by Reprocell), PSGro hESC/iPSC medium (manufactured by System Biosciences), NutriStem (registered trademark) medium (manufactured by Biological Industries), CSTI-7 medium (manufactured by Cell Science Laboratory), MesenPRO RS medium (manufactured by Thermo Fisher Scientific), MF-Media (registered trademark) mesenchymal stem cell growth medium (manufactured by Toyobo Co., Ltd.), Sf-900II (manufactured by Thermo Fisher Scientific), Opti-Pro (manufactured by Thermo Fisher Scientific) are exemplary examples of the basal medium. One kind of these may be used alone and two or more kinds thereof may be used by being mixed.

In addition, examples of the additive to be added to the basal medium include those usually used for culturing nerve cells. Component N (Elixirgen Scientific), Component G2 (Elixirgen Scientific), N2 Supplement (Thermo Fisher Scientific), iCell Neuronal Supplement B (CDI), iCell Neuvous System Supplement, B-27 plus (Thermo Fisher Scientific), and the like are exemplary examples thereof.

### [Method for producing cell-containing container]

The present invention provides a method for producing a cell-containing container according to one embodiment includes a step of incubating a container including nerve cells and a medium, under a culture condition, while replacing the medium at a predetermined timing, in which a concentration of glucose in the medium is maintained at 1 g/L or higher for a predetermined period.

The cell-containing container described above can be produced by the production method of the present embodiment. In addition, as will be described later in Examples, a cell-containing container in which the aggregation of nerve cells is suppressed can be produced by the production method of the present embodiment.

In the production method of the present embodiment, a container (culture container), nerve cells, and a medium are the same as those described above. Specifically, for example, an electrode array may be placed on a culture surface of the cell-containing container. In addition, the nerve cells may be derived from stem cells. Moreover, the stem cells may be human cells.

Replacing 10% to 100% by volume of the total amount of the medium 1 to 10 times a week is an exemplary example of replacing the medium at a predetermined timing. The timing of the medium replacement may be, for example, 1 to 8 times a week, 1 to 5 times a week, or 1 to 3 times a week. It is preferable that the period from the medium replacement to a next medium replacement is substantially constant.

Further, the amount of the medium to be replaced in one medium replacement may be 10% to 80% by volume of the total amount of the medium, 10% to 50% by volume of the total amount of the medium, and 10% to 30% by volume of the total amount of the medium.

In addition, the culture condition may be a condition normally used for culturing nerve cells, conditions of 37°C and 5% CO₂ is an exemplary examples thereof. In addition, an oxygen concentration may be set to 0% to 1%.

An incubation period can be appropriately set according to the purpose. For example, in a case where the action potential is detected in nerve cells that have been induced to differentiate from human iPS cells, the incubation period can be, for example, 30 days or more, for example, 40 days or more, for example, 50 days or more, for example, 60 days or more, and for example, 70 days or more from the time point when the nerve cells dissociated into a single cell are seeded in the culture container.

In the production method of the present embodiment, the concentration of glucose in the medium is maintained at 1 g/L or higher for a predetermined period. Here, the predetermined period may be an entire period in which the nerve cells are incubated under the culture conditions, or may be, for example, at least 20 days, for example, 22 days, and for example, 24 days from the start of the culture. In addition, the concentration of the glucose in the medium is preferably maintained at 0.2 g/L or higher for at least 30 days from the start of the culture, for example, 35 days from the start of the culture, and for example, 40 days from the start of the culture. Here, the start of culture refers to a time point when nerve cells dissociated into a single cell are seeded in a culture container.

In the production method of the present embodiment, the nerve cells adhere the culture surface of the cell-containing container, and an adhesion area between the nerve cells and the culture surface is preferably 0.5 mm² or more, preferably 0.949 mm² or more, more preferably 3 mm² or more, and still more preferably 3.14 mm² or more, per 80,000 nerve cells. Also, an upper limit of the adhesion area between the nerve cells and the culture surface of the culture container is preferably about 28.2 mm².

### [Examples]

Hereinafter, the present invention will be described in more detail with reference to Examples, but the present invention is not limited to the following examples.

### [Experimental Example 1]

### (Examination of changes over time in glucose concentration in medium)

Human nerve cells were cultured. In addition, during the process, a change over time in the glucose concentration in the medium was measured. The cell medium was replaced 3 times a week. In addition, the amount of medium to be replaced was changed for comparison.

As the human nerve cells, Human iPSC-derived GABAergic Neurons (Elixirgen Scientific) was used.

As the cell culture container, a MEA plate (model number "M768-tMEA-48W", Axion Biosystems) having a microelectrode array (MEA) was used. The culture surface of the MEA plate was coated with polyethyleneimine (PEI, Thermo Fisher Scientific) and laminin (Thermo Fisher Scientific). A volume per well of the culture container was 300 µL.

From the day of seeding the nerve cells (0 days in vitro, hereinafter sometimes referred to as "DIV0") to the 5th day (hereinafter, sometimes referred to as "DIV5", and the same applies hereinafter), Quick-Neuron (TM) GABAergic Maintenance Medium" (product name) (Elixirgen Scientific) was used as the medium. The glucose concentration in the medium was 5 g/L. DIV6 and after, "Complete Brainpys Medium" (product name) (CDI) was used as the medium. The glucose concentration in the medium was 0.5 g/L.

The medium was replaced as follows. First, 250 µL, 150 µL or 50 µL of medium was aspirated from the culture container at one time. Subsequently, a new medium having the same capacity as the aspirated medium was added. The medium was replaced three times a week. The aspirated medium was used to measure a glucose concentration. FLEX2 (Nova Biomedical Co., Ltd.), which is a medium component analyzer, was used for measuring the glucose concentration.

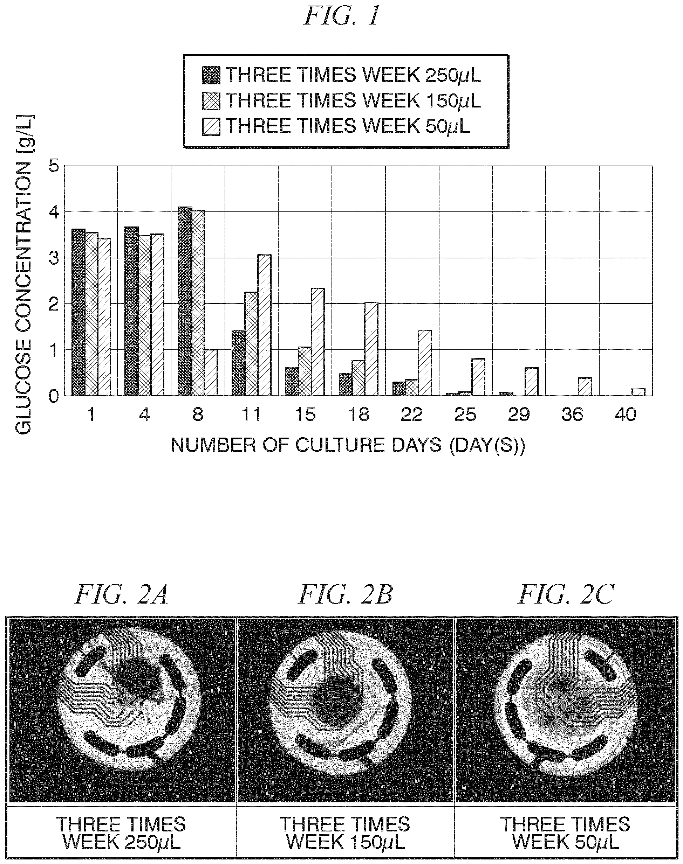

FIG. 1 is a graph showing results of measuring changes over time in glucose concentration in a medium. A horizontal axis shows the number of culture days from seeding of nerve cells, and a vertical axis shows the glucose concentration (g/L) in the medium.

As a result, it was clarified that the glucose concentration in the medium was maintained at the highest level in a case where 50 µL of the medium once was replaced three times a week. When 50 µL of medium once was replaced, the glucose concentration in the medium on the 22nd day after seeding the nerve cells was about 1.4 g/L. In addition, the glucose concentration in the medium on 40th day after seeding the nerve cells was about 0.2 g/L.

On the other hand, in a case where 150 µL of medium once was replaced three times a week, the glucose concentration in the medium on the 22nd day after seeding the nerve cells was about 0.4 g/L. In addition, the glucose concentration in the medium on 40th day after seeding the nerve cells was about 0 g/L.

In addition, in a case where 250 µL of medium once was replaced three times a week, the glucose concentration in the medium on the 22nd day after seeding the nerve cells was about 0.3 g/L. In addition, the glucose concentration in the medium on 40th day after seeding the nerve cells was about 0 g/L.

### [Experimental Example 2]

### (Examination of aggregation level of nerve cells)

Each nerve cell cultured in Experimental Example 1 was observed with a microscope, and an aggregation level thereof was evaluated. Evaluation criteria for the aggregation level were as follows. As the aggregation level increased, the adhesion area between the nerve cells and the culture surface became smaller and the nerve cells tended to be separated from the culture surface.

### «Evaluation criteria for aggregation level»

1: Adhesion area between the nerve cells and the culture surface was 3.14 mm² or more and 28.2 mm² or less per 80,000 nerve cells.
2: Adhesion area between the nerve cells and the culture surface was 0.949 mm² or more and less than 3.14 mm² per 80,000 nerve cells.
3: Adhesion area between the nerve cells and the culture surface was 0.196 mm² or more and less than 0.949 mm² per 80,000 nerve cells.
4: Adhesion area between the nerve cells and the culture surface was 0 mm² or more and less than 0.196 mm² per 80,000 nerve cells.

FIGS. 2A to 2C show representative micrographs of each nerve cell at 53rd day (DIV53) from seeding of nerve cells. FIG. 2A shows a micrograph of nerve cells obtained by replacing 250 µL of the medium once three times a week. FIG. 2B shows a micrograph of nerve cells obtained by replacing 150 µL of the medium once three times a week. FIG. 2C shows a micrograph of nerve cells obtained by replacing 50 µL of the medium once three times a week.

As a result, the aggregation level of the nerve cells on DIV53 obtained by replacing 250 µL of the medium once three times a week was 4. In addition, the aggregation level of the nerve cells on DIV53 obtained by replacing 150 µL of the medium once three times a week was 3. In addition, the aggregation level of the nerve cells on DIV53 obtained by replacing 50 µL of the medium once three times a week was 1.

From the results, it was clarified that the nerve cells cultured under the condition in which the glucose concentration in the medium was maintained high tended to have a low aggregation level. More specifically, it was clarified that the nerve cells having a glucose concentration of 1 g/L or higher in the medium on 22nd day after seeding the nerve cells tended to have a low aggregation level. Furthermore, it was clarified that the nerve cells having a glucose concentration of 0.2 g/L or higher in the medium on 40th day after seeding the nerve cells tended to have a low aggregation level.

### [Experimental Example 3]

### (Examination of aggregation level and action potential of nerve cells)

In the same manner as Experimental Example 1, nerve cells cultured for 42 days from seeding of the nerve cells was observed with a microscope, and an aggregation level thereof was evaluated. Evaluation criteria for the aggregation level were the same as those of Experimental Example 2. In addition, the action potential of each nerve cell was measured and evaluated using a microelectrode array. The evaluation criteria of the action potential were as follows, and detectability for the action potential could be detected was determined.

### «Evaluation criteria for action potential»

A: The action potential could be detected well.
B: The action potential could be detected.
C: The action potential could not be detected.

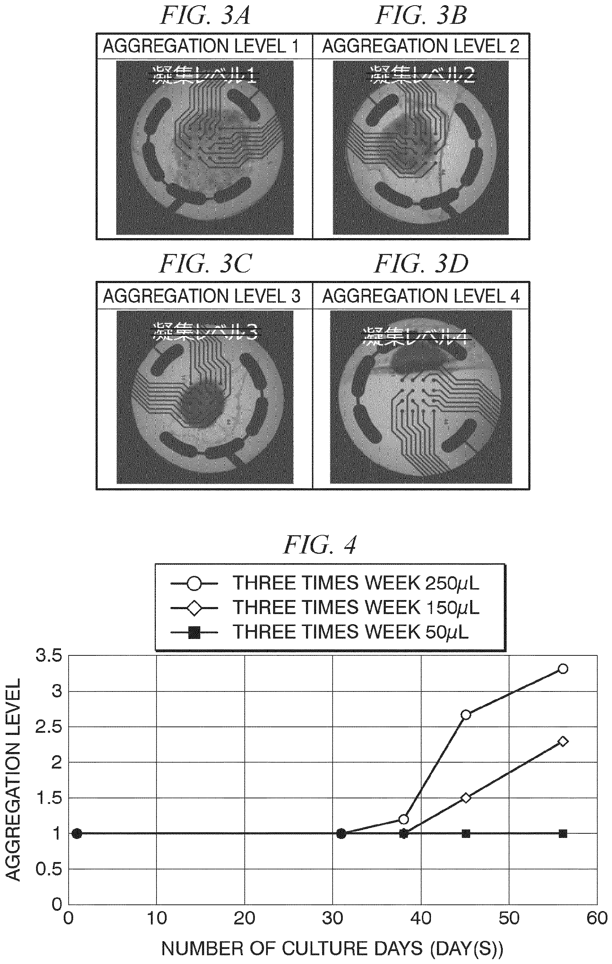

FIG. 3A shows a representative micrograph of nerve cells evaluated as Aggregation Level 1. FIG. 3B shows a representative micrograph of nerve cells evaluated as Aggregation Level 2. FIG. 3C shows a representative micrograph of nerve cells evaluated as Aggregation Level 3. FIG. 3D shows a representative micrograph of nerve cells evaluated as Aggregation Level 4. In addition, Table 1 below shows evaluation results of the aggregation level and the action potential.

**[Table 1]**

| | | | | |
|---|---|---|---|---|
| Aggregation level | 1 | 2 | 3 | 4 |
| Determination of detectability for action potential | A | B | C | C |

As a result, it was clarified that when nerve cells evaluated as Aggregation Level 1 are used, the action potential can be detected well.

### [Experimental Example 4]

### (Examination of change over time in aggregation level of nerve cells)

The change over time in the aggregation level of each nerve cell cultured in the same manner as in Experimental Example 1 was observed. Evaluation criteria for the aggregation level were the same as those of Experimental Example 2.

FIG. 4 is a graph showing changes over time in the aggregation level of nerve cells obtained by replacing 250 µL, 150 µL, or 50 µL of a medium once three times a week. As a result, it was clarified that the nerve cells obtained by replacing 50 µL of a medium once three times a week maintained Aggregation Level 1 even on 56th day after the seeding the nerve cells.

On the other hand, it was recognized that the aggregation level of the nerve cells obtained by replacing 150 µL of a medium once three times a week increase after 38th day after seeding the nerve cells. In addition, it was recognized that the aggregation level of the nerve cells obtained by replacing 250 µL of a medium once three times a week increase after 31st day after seeding the nerve cells.

The results indicate that the lower the glucose concentration in the medium, the higher the aggregation level of the nerve cells tends to be. In the related art, a culture condition of nerve cells obtained by replacing 150 µL of a medium once three times a week is generally adopted. On the other hand, it was clarified that in a case of the culture condition of the nerve cells obtained by replacing 50 µL of a medium once three times a week, the aggregation level of the nerve cells can be maintained low.

### [Experimental Example 5]

### (Examination of relationship between glucose concentration in medium and aggregation level of nerve cells)

In the same manner as in Experimental Example 1, nerve cells were cultured by varying the amount of medium replaced. Moreover, relationships between the glucose concentration of the nerve cells in the medium on 22nd day (DIV22) from the seeding of the nerve cells and aggregation levels of the nerve cells on the 31st day (DIV31), the 38th day (DIV38), the 45th day (DIV45), and 56th day (DIV56) from the seeding of the nerve cells were examined.

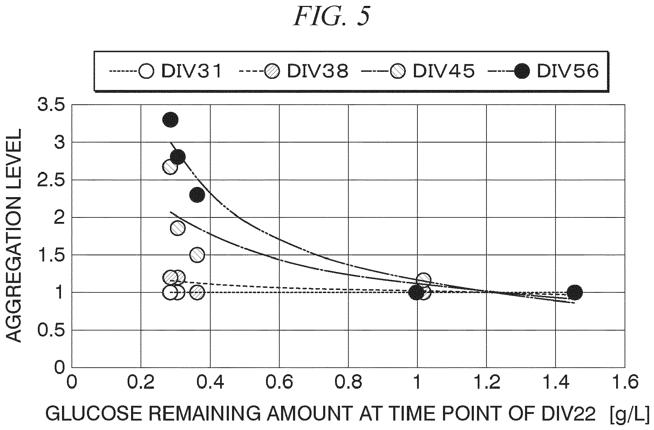

FIG. 5 is a graph showing examination results. In FIG. 5, a horizontal axis shows the glucose concentration (g/L) of the nerve cells in the medium on DIV22, and a vertical axis shows the aggregation level of the nerve cells evaluated by the same evaluation criteria as in Experimental Example 2.

As a result, it was clarified that when the glucose concentration of the nerve cells in the medium on 22nd day (DIV22) from the seeding of the nerve cells is 1 g/L or higher, the aggregation levels of the nerve cells even on the 31st day (DIV31), the 38th day (DIV38), the 45th day (DIV45), and 56th day (DIV56) tended to be maintained low.

On the other hand, it was clarified that when the glucose concentration of the nerve cells in the medium on 22nd day (DIV22) from the seeding of the nerve cells is less than 1 g/L, the aggregation levels of the nerve cells on the 31st day (DIV31), the 38th day (DIV38), the 45th day (DIV45), and 56th day (DIV56) tended to hardly increase.

The present invention includes the following aspects.
[1] A cell-containing container including:
   nerve cells; and
   a medium,
   in which the nerve cells adhere to a culture surface of the cell-containing container,
   an adhesion area between the nerve cells and the culture surface is 0.5 mm² or more per 80,000 nerve cells, and
   a concentration of glucose in the medium is 1 g/L or higher.
[2] The cell-containing container according to [1],
   in which an electrode array is placed on the culture surface.
[3] The cell-containing container according to [1] or [2],
   in which the nerve cells are derived from stem cells.
[4] The cell-containing container according to [3],
   in which the stem cells are human cells.
[5] A method for producing a cell-containing container, the method including:
   incubating a container including nerve cells and a medium, under a culture condition, while replacing the medium at a predetermined timing,
   in which a concentration of glucose in the medium is maintained at 1 g/L or higher for a predetermined period.
[6] The production method according to [5],
   in which the incubating is performed for 30 days or longer.
[7] The production method according to [5] or [6],
   in which the nerve cells adhere to a culture surface of the cell-containing container, and
   an adhesion area between the nerve cells and the culture surface is 3 mm² or more per 80,000 nerve cells.
[8] The production method according to [7],
   in which an electrode array is placed on the culture surface.
[9] The production method according to any one of [5] to [8],
   in which the nerve cell are derived from stem cells.
[10] The production method according to [9],
   in which the stem cells are human cells.

While preferred embodiments of the invention have been described and illustrated above, it should be understood that these are exemplary of the invention and are not to be considered as limiting. Additions, omissions, substitutions, and other modifications can be made without departing from the scope of the invention. Accordingly, the invention is not to be considered as being limited by the foregoing description and is only limited by the scope of the appended claims.

## Claims

1. A cell-containing container comprising:
nerve cells; and
a medium,
wherein the nerve cells adhere to a culture surface of the cell-containing container,
an adhesion area between the nerve cells and the culture surface is 0.5 mm² or more per 80,000 nerve cells, and
a concentration of glucose in the medium is 1 g/L or higher.

2. The cell-containing container according to Claim 1,
wherein an electrode array is placed on the culture surface.

3. The cell-containing container according to Claim 1 or 2,
wherein the nerve cells are derived from stem cells.

4. The cell-containing container according to Claim 3,
wherein the stem cells are human cells.

5. A method for producing a cell-containing container, the method comprising:
incubating a container including nerve cells and a medium, under a culture condition, while replacing the medium at a predetermined timing,
wherein a concentration of glucose in the medium is maintained at 1 g/L or higher for a predetermined period.

6. The production method according to Claim 5,
wherein the incubating is performed for 30 days or longer.

7. The production method according to Claim 5 or 6,
wherein the nerve cells adhere to a culture surface of the cell-containing container, and
an adhesion area between the nerve cells and the culture surface is 3 mm² or more per 80,000 nerve cells.

8. The production method according to Claim 7,
wherein an electrode array is placed on the culture surface.

9. The production method according to any one of Claims 5 to 8,
wherein the nerve cell are derived from stem cells.

10. The production method according to claim 9,
wherein the stem cells are human cells.
